# EUROPEAN PATENT SPECIFICATION

(11) **EP 0 470 665 B1**
(45) Date of publication and mention of the grant of the patent: **24.04.1996**
(21) Application number: 91201994.0
(22) Date of filing: 01.08.1991
(51) Int. Cl.: A01N 43/38, A01N 43/40, C07D 209/42, C07D 209/30

(54) **1-Hydroxyindole fungicides**
Fungizide 1-Hydroxyindole
Fongicides 1-hydroxyindoliques

(30) Priority: 06.08.1990 US 562998
(43) Date of publication of application: 12.02.1992
(73) Proprietor: EASTMAN KODAK COMPANY, Rochester, New York 14650-2201 (US)
(72) Inventor: Schleigh, William Robert, c/o Eastman Kodak Co., Rochester, New York 14650-2201 (US); Welter, Thomas Robert, c/o Eastman Kodak Co., Rochester, New York 14650-2201 (US)
(74) Representative: Nunney, Ronald Frederick Adolphe

(56) References cited:
- FR-A- 1 497 492
- US-A- 3 296 277

## Description

The present invention relates to fungicides.

In view of world hunger, it is useful to provide the public with a variety of fungicides for use in food agriculture. While a number of fungicides are known in the art, alternative fungicides are needed.

The general structure of 1-hydroxyindoles is known in the art. 2-substituted-1-hydroxyindoles have been disclosed in two references. US-A-3,296,277 teaches a 3-cyano-1-hydroxy-2-phenylindole having the phenyl group bearing a nitro or a lower alkoxy substituent. As disclosed, the use of the 2-substituted -1-hydroxyindole was pharmacological in nature, acting on the central nervous system as a depressant or as an adrenolytic agent. Loudon, et al., Journal of the Chemical Society, 3466 (1960) teach the preparation of a 3-cyano-1-hydroxy-2-phenylindole without disclosing a use for the compound.

It is known in the art to employ N-substituted indoles as fungicides. For instance, FR-A-1 497 492 discloses N-sulfenyl indoles for controlling fungus. A N-substituted indole with an oxygen substituent on the 1-position of the indole, however, has not been described as an active fungicide.

Providing the public with a novel process for controlling fungi is desirable. A new process would offer more flexibility and choice to users of known fungicides.

An alternative process for controlling fungi is now provided comprising contacting the fungi with an active compound characterized wherein the compound is employed in an amount ranging from 1 to 90 weight percent and is a 1-hydroxyindole having a formula as follows: 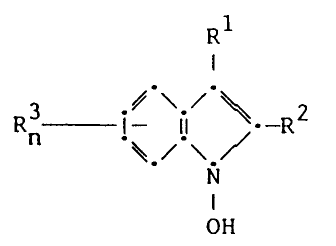
wherein
R¹ is hydrogen,carbamoyl, t-butylcarbamoyl, dimethylcarbamoyl, carboxy, nitro or cyano,
R² is an alkenyl having 2-10 carbon atoms, a N-substiiuted-α-iminobenzyl, an unsubstiiuted or substituted aromatic group, or an acyl having 2-16 carbon atoms,
R³ is a halogen atom, and
n is an integer from 0 to 4.

In another aspect of the invention, a compound useful in the process as described above is provided comprising the structure as shown above
wherein
R¹ is cyano or hydrogen and R², R³, and n are as defined above.

Foliar phytopathogenic fungi are controlled by applying a fungicidally effective amount of compounds of a formula 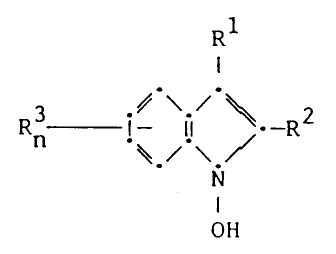
wherein
R¹ is hydrogen or an electron withdrawing group selected from the group consisting of carbamoyl, t-butylcarbamoyl and dimethylcarbamoyl, carboxy, nitro, cyano,
R² is an alkenyl having 2-10 carbon atoms, such as vinyl, allyl or butenyl, N-substituted-α-iminobenzyl groups, that is, wherein the imino nitrogen is substituted, preferably with a phenyl, anilino or dimethylamino group, for example, α-(phenylimino)benzyl, α-(anilinoimino)benzyl and α-(dimethylaminoimino)-benzyl, an unsubstituted or substituted aromatic group having 5-6 nuclear atoms in the aromatic ring, such as phenyl, nitrophenyl, trifluoromethylphenyl, tolyl, 4-methoxyphenyl, 4-phenylsulfonylphenyl, 4-benzophenonyl, 4-t-butylphenyl, chlorophenyl, 4-(2-cyanovinyl)phenyl, bromophenyl, 4-(2-carboxyvinyl)phenyl, dichlorophenyl, fluorophenyl, formylphenyl, hydroximinomethylphenyl, carboxymethylphenyl, carboxyphenyl, hydroxyphenyl, sulfamoylphenyl, acetylphenyl, cyanophenyl, 2-furyl, 4-carbamoylphenyl, 2-furanyl, 4-t-butylphenyl, pyridyl, dimethoxyphenyl, and so on, and acyl having 2-16 carbon atoms, for example, alkylcarbonyl wherein the alkyl group has about 1-16 carbon atoms, or arylcarbonyl wherein said aryl group has about 5-16 carbon atoms, for example, 2,2-dimethylpropionyl (that is, neopentanoyl), acetyl, butyryl, octanoyl, benzoyl, 3,4-dichlorobenzoyl, 4-methylbenzoyl, 4-bromobenzoyl, 3-trifluoromethylbenzoyl, chlorobenzoyl, 3,4-dimethoxybenzoyl, 4-methoxybenzoyl, and so on,
R³ is a halogen atom, such as chloro, bromo, iodo, or fluoro, and
n is an integer from 0 to 4 and it is understood that when n is less than 4, hydrogen fills the unsubstituted positions.

Preferred methods of the invention utilize compounds having the above structure wherein
R¹ is cyano or hydrogen and R², R³ and n are as described above.

Other preferred methods of the invention utilize compounds having the above structure wherein
R¹ is cyano,
R² is 4-nitrophenyl, 3-nitrophenyl, 3-trifluoromethylphenyl, 4-methoxyphenyl, 4-fluorophenyl, 4-formylphenyl, 4-carbamoylphenyl, 2-furyl, vinyl, 4-pyridyl, 2-pyridyl, phenyl, 3,4-dichlorophenyl, 4-methylphenyl, 4-bromophenyl, 4-chlorophenyl, 3,4-dimethoxyphenyl, 4-methoxyphenyl, 3-trifluoromethylbenzoyl, 4-t-butylphenyl, hydroxyiminomethylphenyl, or α-(phenylimino)benzyl and
n is 0 and represents no substituents at R³.

Further preferred methods of the invention utilize compounds having the above structure wherein
R¹ is cyano,
R² is an alkenyl having 2-10 carbon atoms, N-substituted-α-iminobenzyl, an unsubstituted or substituted aromatic group and acyl having 2-16 carbon atoms, and
n is 0,
or
R¹ is cyano,
R² is phenyl or 4-nitrophenyl, and
n is 0,
or
R¹ is hydrogen,
R² is phenyl, 4-nitrophenyl or
4-chlorophenyl,
R³ is chloro, and
n is from 0 to 2.

The most preferred methods of the invention utilize compounds having the above structure wherein:
R² is a para- or meta- substituted phenyl group, preferably 3-nitrophenyl or 4-nitrophenyl.

Further, a fungicidal composition is provided comprising a fungicidally effective amount of at least one of the following active ingredients:
3-cyano-1-hydroxy-2-(4-nitrophenyl)indole,
3-cyano-1-hydroxy-2-phenylindole,
3-cyano-1-hydroxy-2-(3-nitrophenyl)indole,
3-cyano-1-hydroxy-2-(4-trifluoromethylphenyl)indole,
3-cyano-1-hydroxy-2-(3-trifluoromethylphenyl)indole,
3-cyano-1-hydroxy-2-(4-methoxyphenyl)indole,
3-cyano-1-hydroxy-2-(4-benzoylphenyl)indole,
3-cyano-1-hydroxy-2-[4-(2-cyanovinyl)phenyl]indole,
3-cyano-2-(3,4-dichlorophenyl)-1-hydroxyindole,
3-cyano-5,6-dichloro-1-hydroxy-2-(4-nitrophenyl)indole,
3-cyano-6-chloro-1-hydroxy-2-(4-nitrophenyl)indole,
3-cyano-5,6-dichloro-1-hydroxy-2-phenylindole,
3-cyano-6-chloro-1-hydroxy-2-phenylindole,
3-cyano-2-(2-furyl)-1-hydroxyindole,
3-cyano-2-vinyl-1-hydroxyindole,
3-cyano-1-hydroxy-2-(4-pyridyl)indole,
3-cyano-1-hydroxy-2-(2-pyridyl)indole,
1-hydroxy-2-phenylindole,
6-chloro-1-hydroxy-2-(4-chlorophenyl)indole,
2-benzoyl-3-cyano-1-hydroxylindole,
3-cyano-2-(3,4-dichlorobenzoyl)-1-hydroxyindole,
3-cyano-1-hydroxy-2-(p-toluoyl)indole,
2-(4-bromobenzoyl)-3-cyano-1-hydroxyindole,
3-cyano-1-hydroxy-2-(3-trifluoromethylbenzoyl)indole,
2-(4-chlorobenzoyl)-3-cyano-1-hydroxyindole,
3-cyano-2-(3,4-dimethoxybenzoyl)-1-hydroxyindole,
3-cyano-1-hydroxy-2-(4-methoxybenzoyl)indole,
2-benzoyl-5-chloro-3-cyano-1-hydroxyindole,
3-cyano-1-hydroxy-2-neopentanoylindole,
3-cyano-2-(4-fluorophenyl)-1-hydroxyindole,
3-cyano-2-(4-formylphenyl)-1-hydroxyindole,
3-cyano-1-hydroxy-2-(α-phenyliminobenzyl)indole,
3-cyano-1-hydroxy-2-(α-phenylazino)indole and
3-cyano-2-(α-dimethylazinobenzyl)-1-hydroxyindole.

Typical compounds representative of those useful in the inventive process include the compounds listed above as active ingredients in the fungicidal compositions of the invention.

The present invention provides a means for controlling wheat leaf rust and other fungi.

The N-hydroxyindoles are generally obtainable as colorless to yellow crystalline materials having characteristic melting points and absorption spectra and which may be purified by recrystallization from common organic solvents. They are appreciably soluble in many organic solvents such as methanol, ethanol, acetone, chloroform, benzene, dioxane, dimethyl sulfoxide and N,N-dimethylformamide, but are relatively insoluble in water.

The compounds of the invention can be prepared, in general, through minor modifications of literature procedures. 2-Chloronitrobenzene can be condensed with ethyl cyanoacetate in the presence of excess potassium hydroxide affording a good yield of the ethyl 2-cyano-2-nitrophenylacetate as can be seen by the following reaction scheme: 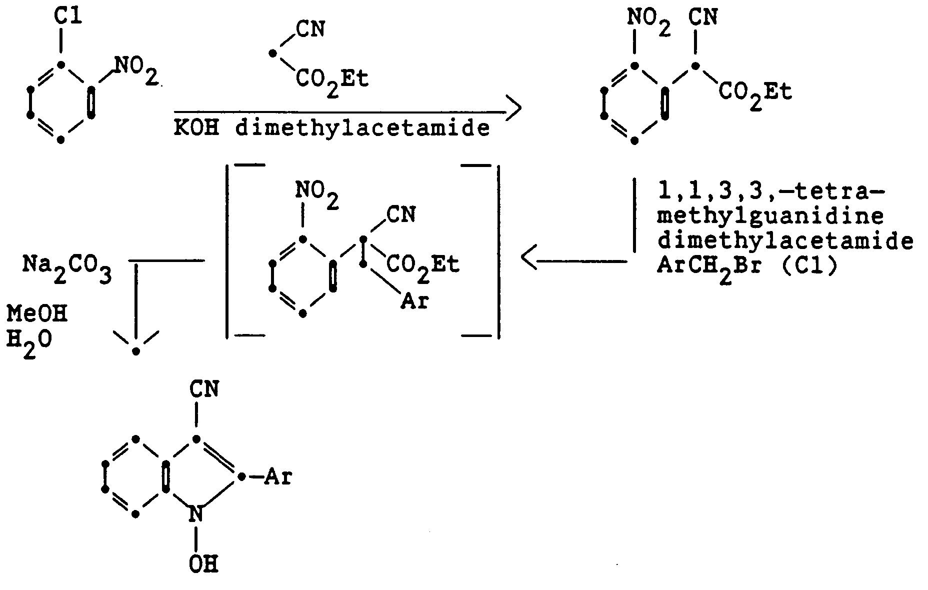
Various benzyl halides can then be condensed with the acetate ester followed by cyclization to the aryl-substituted indole as shown. Bromides are preferred.

A series of 2-keto derivatives were also prepared wherein:
N-hydroxy-2-benzoyl-3-cyanoindole was prepared by cyano addition-cyclization of 1-(2-nitrophenyl)-3-phenyl-1-propen-3-one (2-nitrochalcone). Various 2-nitrochalcones could be prepared via condensation of 2-nitrobenzaldehydes with acetophenones. Thus, the series of 2-keto derivatives was prepared as exemplified by the following scheme: 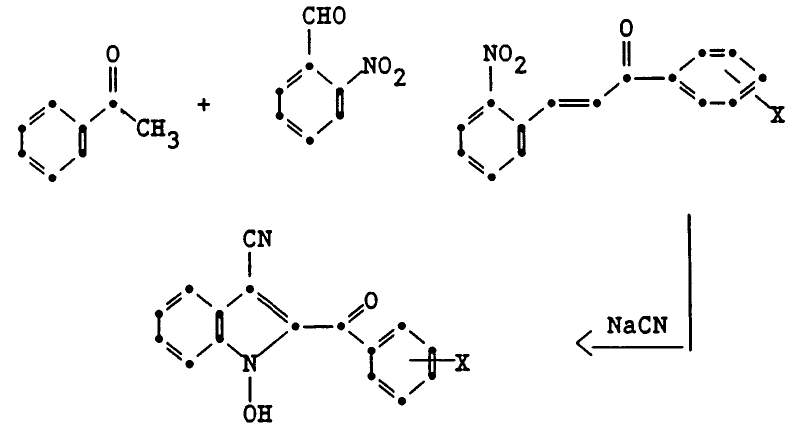
The preferred 2-acyl compounds useful in the process of this invention are
2-benzoyl-3-cyano-1-hydroxyindole;
3-cyano-2-(3,4-dichlorobenzoyl)-1-hydroxyindole;
3-cyano-1-hydroxy-2-(p-toluoyl)indole;
2-(4-bromobenzoyl)-3-cyano-1-hydroxyindole; and
2-(4-chlorobenzoyl)-3-cyano-1-hydroxyindole.

The 1-hydroxyindoles used in the invention can be applied as fungicidal sprays by methods commonly employed at varying concentrations, air-blast, aerial sprays and dusts. The dilution and rate of application will depend upon the type of equipment employed, the method and frequency of application desired and diseases to be controlled.

Such active compounds may be employed alone or in the form of mixtures with such solid and/or liquid carrier vehicles and/or with other known compatible active agents, especially plant protection agents, such as other insecticides, nematocides, or acaricides, fungicides, bactericides, rodenticides, herbicides, fertilizers, growth-regulating agents, and so on, if desired, or in the form of particular dosage preparations for specific application made therefrom, such as solutions, emulsions, suspensions, powders, pastes, and granules which are thus ready for use.

The process of the present invention is useful for the control of wheat leaf rust and can be utilized on the foliage. For such purposes, these compounds can be used in solutions, liquid formulations, or dry powder formulations. The compounds are usually taken up in a carrier or are formulated so as to render them suitable for subsequent use as fungicides. For example, these chemical agents can be formulated as wettable powders, dry powders, emulsifiable concentrates, dusts, granular formulations, aerosols, or flowable emulsion concentrates. In such formulations, the compounds are extended with a liquid or solid carrier and, when dried, suitable surfactants are incorporated.

It is usually desirable, particularly in the case of foliar spray formulations, to include adjuvants, such as wetting agents, spreading agents, dispersing agents, stickers, adhesives and the like in accordance with agricultural practices.

In general, the compounds utilized in this invention can be dissolved in appropriate solvents such as acetone, methanol, ethanol, dimethylformamide or methyl sulfoxide and such solutions extended with water. The concentration of the solution can vary from 1 to 90% (with all percentages hereinafter defined as the weight percentage of active compound). The preferred range is from 5 to 50%.

For the preparation of emulsifiable concentrates, the compounds used in the invention can be dissolved in suitable organic solvents or a mixture of solvents, together with an emulsifying agent which permits dispersion of the fungicide in water. The concentration of the active ingredient in emulsifiable concentrates is usually 10 to 90% and in flowable emulsion concentrates, this can be as high as 75%.

Wettable powders suitable for spraying, can be prepared by admixing the compound with a finely divided solid, such as clays, inorganic silicates and carbonates, and silicas and incorporating wetting agents, sticking agents, and/or dispersing agents in such mixtures. The concentration of active ingredients in such formulations varies widely.

Dusts are prepared by mixing the 1-hydroxyindoles and salts and complexes thereof with finely divided inert solids which can be organic or inorganic in nature. Materials useful for this purpose include botanical flours, silicas, silicates, carbonates and clays. One convenient method of preparing a dust is to dilute a wettable powder with a finely divided carrier. Dust concentrations containing 20% to 80% of the active ingredient are commonly made and are subsequently diluted to 1% to 10% use concentration.

The compounds of the invention were particularly tested for activity against Colletotrichum lagenarium (Anthracnose on cucumbers), Puccinia recondita (wheat leaf rust), Erisiphe polygoni (powdery mildew on beans), Phytophthora infestans (late blight on tomatoes), Rhizoctonia solani (Rhizoctonia on cotton), Pythium sp. (damping off on peas), and Botrytis cinerea (gray mold). They showed particularly enhanced activity against rust disease, including Puccinia recondita (wheat leaf rust).

### Example I:

The compounds used in this invention were evaluated as protectant fungicides by standard methods A and B. The results of these primary evaluations are recorded in Table I and some in Table V. Furthermore, certain of the compounds were evaluated for their dose response profiles against wheat leaf rust. The procedure was the same as procedure A with the exception that various levels of test compound were applied as described in Step 18.
- METHOD A -: Evaluation of Test Compounds for Control of Foliar Pathogens
Five pathogens were used for evaluating potential fungicides. Refer to Table A-1 for specific protocol of each test.
1. Host plants were grown in 13.3 x 13.3 cm, units held in standard greenhouse flats (52 x 26 x 6 cm). The soil used was steam-sterilized sandy loam. Plants were grown to the appropriate stage and then treated with the test chemical.
2. Ten ml of the test solution containing 500 ppm active ingredient per ml was applied per unit sample. The appropriate amounts of chemical per unit was calculated as follows:$\frac{\text{0.05 x 10}}{\text{100}} \text{= grams of chemical per 13.3x 13.3 x 6 cm unit}$ where,
.05 = 500 parts per million (hereinafter ppm) converted to percent, 10 = ml of diluent, and 100 = percent active ingredient of chemical sample, in this case, it was 100% or technical grade.
3. A solution of an octylphenoxy polyethoxy ethanol non-ionic surfactant sold by Rohm & Haas Co. as Triton™ X-100 in acetone (1000 ppm weight per volume) was used to dissolve the material under test. The solution was diluted with distilled water 1:9 volume/volume to obtain a final mixture of 10% acetone and 100 ppm of Triton™ X-100 non-ionic surfactant in water. Further
dilution of this stock solution, as required in rate studies, was done by using a diluent consisting of 100 ppm Triton™ X-100 non-ionic surfactant in water so that a constant concentration of the surfactant was maintained at all levels.
4. The test consists of:
A. Untreated control
B. Test
C. Standard

5. The standards (Table A-I) were applied at the given rate. Some lesions may have occurred at these rates.
6. Two replicates per test.
7. Test compounds were applied at 1.4 kgf/cm² using a hand-held spray gun. The plants were rotated on a turn table during application to assure even coverage.
8. Following spraying, the foliage was air dried.
9. Bean plants were then placed adjacent to mildew-infested beans. After 24 hours exposure, the plants were moved to the opposite end of the greenhouse and held until mildew first appeared on the control. The test compound was then evaluated. The plants were further held until 100% leaf infection occurred in the control, and a final readout were taken.
10. The remaining test plants: tomatoes, cucumbers and wheat were inoculated with the respective pathogen (Table A-I). Spores were obtained from culture plates and diluted in 1% glucose plus 1 drop of polyoxyethylene(20) sorbitan monolaurate(Tween™20) per 100 ml of solution. Spores were sprayed over the plant foliage at 10 ψ using a spray atomizer. Fifteen ml of spray was applied per greenhouse flat (8 units).
11. Following inoculation, the plants were placed into an incubation chamber for 48 hours. Tomatoes inoculated with P. infestans were held at 20°C and 100% relative humidity. All others were held at 25°C and 100% relative humidity. The humidity was maintained by an overhead sprayer which produced fine mist for 15 seconds every 15 minutes.
12. Following incubation, the plants were placed on a greenhouse bench. An overhead misting unit continued to wet the foliage for 15 seconds every 15 minutes.
13. The activity of the test compound was then evaluated when lesions first appeared in the control, generally in 2 to 3 days. The plants were then held until 100% leaf infection occurred in the control, and a final readout was taken.
14. The following information was recorded:
A. Number of healthy plants
B. Number of diseased plants
C. Number of lesions
D. Phytotoxicity, that is, chlorosis, marginal leaf burning, stunting, unusual growth patterns, and so on.

15. The percentage disease control was calculated according to the following formula:$\text{MPDC =} \frac{\text{MDIC - MDIT}}{\text{MDIC}} \text{x 100}$ where,
- MPDC =: mean percentage of disease control,
- MDIC =: mean percentage of disease incidence in the untreated control,
and
- MDIT =: mean percentage of disease incidence in the treatment.

16. Based on the percentage of disease control, treatments are ranked 0 to 4 using the following scale:

| % Control | Ranking |
|---|---|
| 0 - 9 | 0 |
| 10 - 29 | 1 |
| 30 - 49 | 2 |
| 50 - 79 | 3 |
| 80 - 100 | 4 |

These primary screening results were recorded in Table I.
17. Compounds which facilitated 50% or better control (ranking of 3 or 4) were subjected to secondary screening.
18. Secondary screening consisted of rate studies using 250, 500, 1000 and 2000 ppm for compounds ranked as 3. Those ranked as 4 are tested at 125, 250, 500 and 1000 ppm. Occasionally, compounds with a 2 ranking were tested at 500, 1000, 2000 and 4000 ppm if the few healthy plants were relatively disease-free. From these data were determined and reported the EC_{50's}, that is, the concentrations at which 50% control of the fungus was observed. These secondary screening results are reported in Tables II to V for wheat leaf rust.

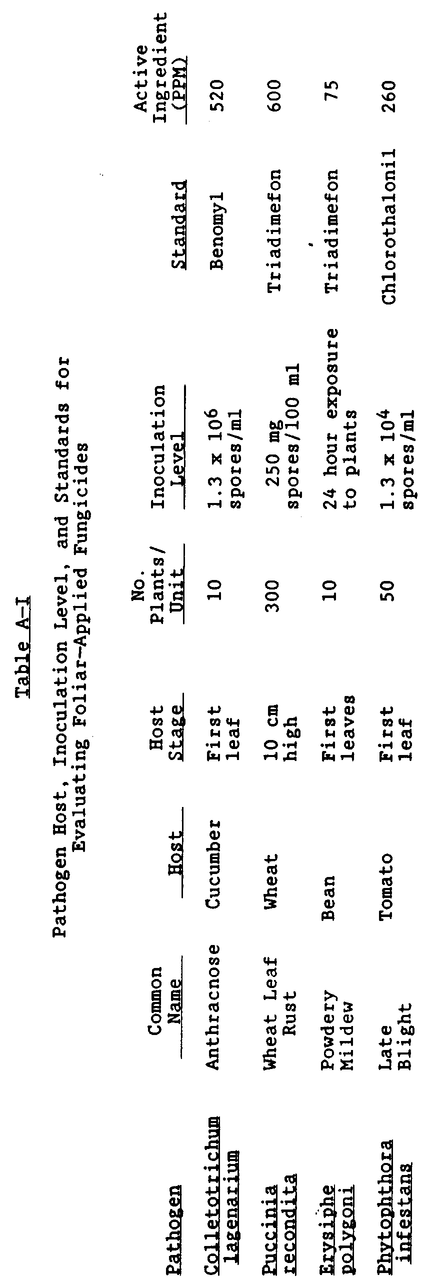

### METHOD B - Evaluation of test compounds for control of RHIZOCTONIA solani

1. Sandy loam soil was steam-sterilized at 190°F (87.8°C) for 48 hours. The soil was then removed and allowed to cool. After cooling 24 hours, the soil generally had a moisture content of 5 to 8 percent.
2. Soil was mixed to insure uniform composition and moisture.
3. Three 150 gram samples of soil were removed, weighed and placed into an oven at 100°F (27.8°C) and allowed to dry for one hour. The approximate percent dry weight was then calculated. This calibration was used to estimate the dry weight of the soil lot.
4. One gallon (approximately 4293.2 grams fresh wt.) of soil was mixed with 3 core samples from Rhizoctonia culture plate. A core sample was a 3.0 cm diameter disc of spores, mycelium, mixed in vermiculite. It was cut using the mount of a test tube. The sample weighed about 3.2 grams. Samples were taken from the margins of fungal growth from plates 4 to 6 weeks old.
5. The mycelium and spores were uniformly distributed throughout the soil sample by hand mixing the soil for 5 minutes.
6. One hundred and fifty grams dry weight (equivalent) of soil were then placed into 10 ounce styrofoam cups.
7. Ten milliliters of test solution containing 50 ppm weight per volume was added to the soil sample. The appropriate amount of chemical per container
was calculated as follows:$\frac{\text{.005 x 150}}{\text{100}} \text{= grams of chemical per 13.3 x 13.3 x 6 cm unit}$ where,

- .005 =: 50 ppm converted to percent
- 150 =: dry weight of soil sample, and
- 100 =: percent active ingredient of chemical sample.

8. A solution of Triton™ X-100 non-ionic surfactant in acetone (1000 ppm weight per weight) is used to dissolve the material under test and the solution was diluted with distilled water 1:9 volume per volume to obtain a mixture of 10 percent acetone and 100 ppm of Triton™ X-100 nonionic surfactant in water. Further dilution of this stock solution, as required in rate studies, was done by using a diluent consisting of 100 ppm Triton X-100 nonionic surfactant in water so that a constant concentration of the surfactant was maintained at all levels.
9. A test consisted of:
A. Control - seed only
B. Control - seed plus inoculum
C. Test - seed plus compound
D. Test - seed plus compound plus inoculum
E. Standard

10. The standard, Tersan™SP (chloroneb), was tested at 50 ppm active ingredient weight per weight.
11. Two replicates of each test were conducted.
12. Following application of the chemical, the cups were capped and vigorously shaken to mix the chemical throughout the soil sample.
13. A tablespoon of soil was then temporarily removed and 10 cotton seeds planted beneath. The soil surface was tamped smooth. Ten milliliters of water were added to the surface and the cup again sealed.
14. The sealed cups were held at 70°F (21.1°C) for 3 days to allow germination. The cups were then uncapped and placed under fluorescent lights for an additional 11 days.
15. The plants were uprooted and examined. If the primary root had one or more lesions, the plant was considered diseased. The following information was recorded:
A. Number healthy plants
B. Number diseased plants
C. Phytotoxicity, that is, chlorosis, root stunting, unusual growth patterns, and so on.

16. The percent healthy plants in each treatment was adjusted to account for natural mortality using Abbott's formula:$\frac{\text{NHC - NHT}}{\text{NHC}} \text{x 100 = APM}$ where,
- APM =: adjusted percentage mortality,
- NAC =: number healthy plants in control (no inoculum, no fungicide), and
- NHT =: number healthy plants in treatment.

17. The percentage disease control was calculated according to the following formula:$\frac{\text{DIC - DIT}}{\text{DIC}} \text{x 100 = PDC}$ where,
- PDC =: percentage of disease control
- DIC =: disease incidence in control (no fungicide), and
- DIT =: disease incidence in treatment.

18. Based on the percentage of disease control, treatments were ranked 0 to 4 using the following scale:

| % Control | Ranking |
|---|---|
| 0 - 9 | 0 |
| 10 - 29 | 1 |
| 30 - 49 | 2 |
| 50 - 79 | 3 |
| 80 - 100 | 4 |

The primary test results are recorded in Table I.

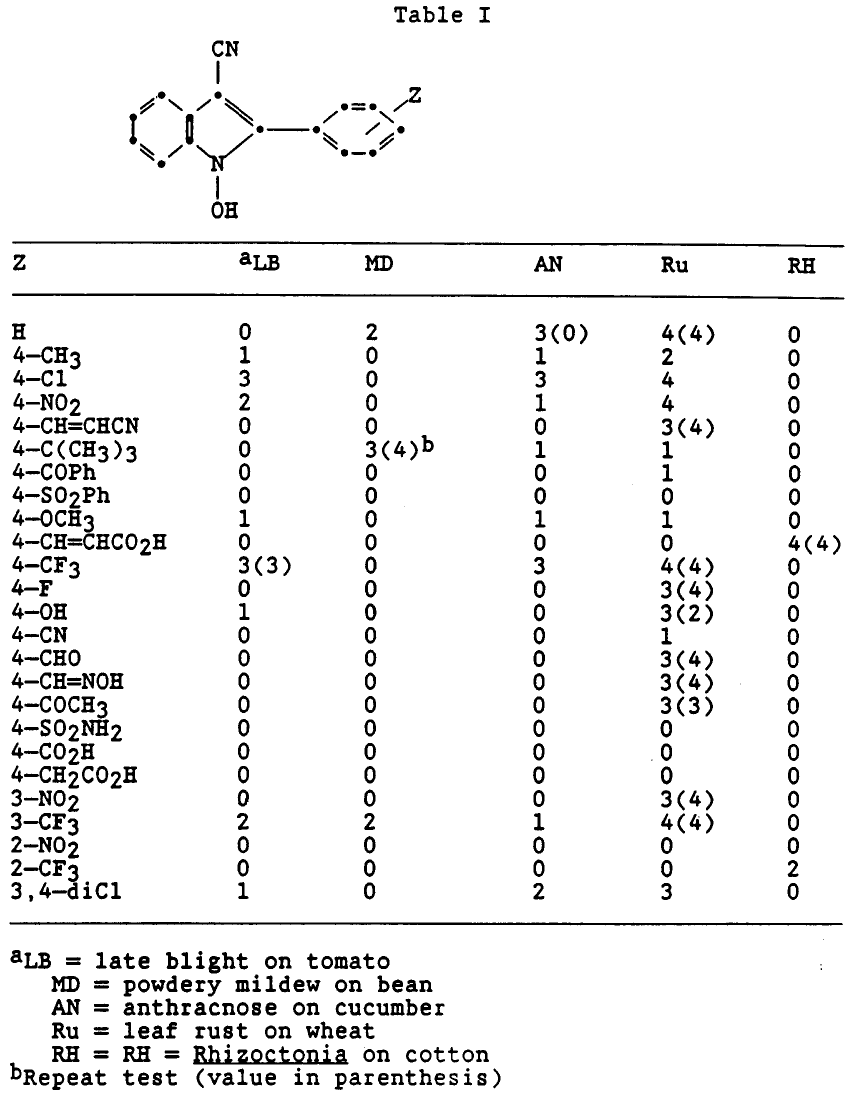

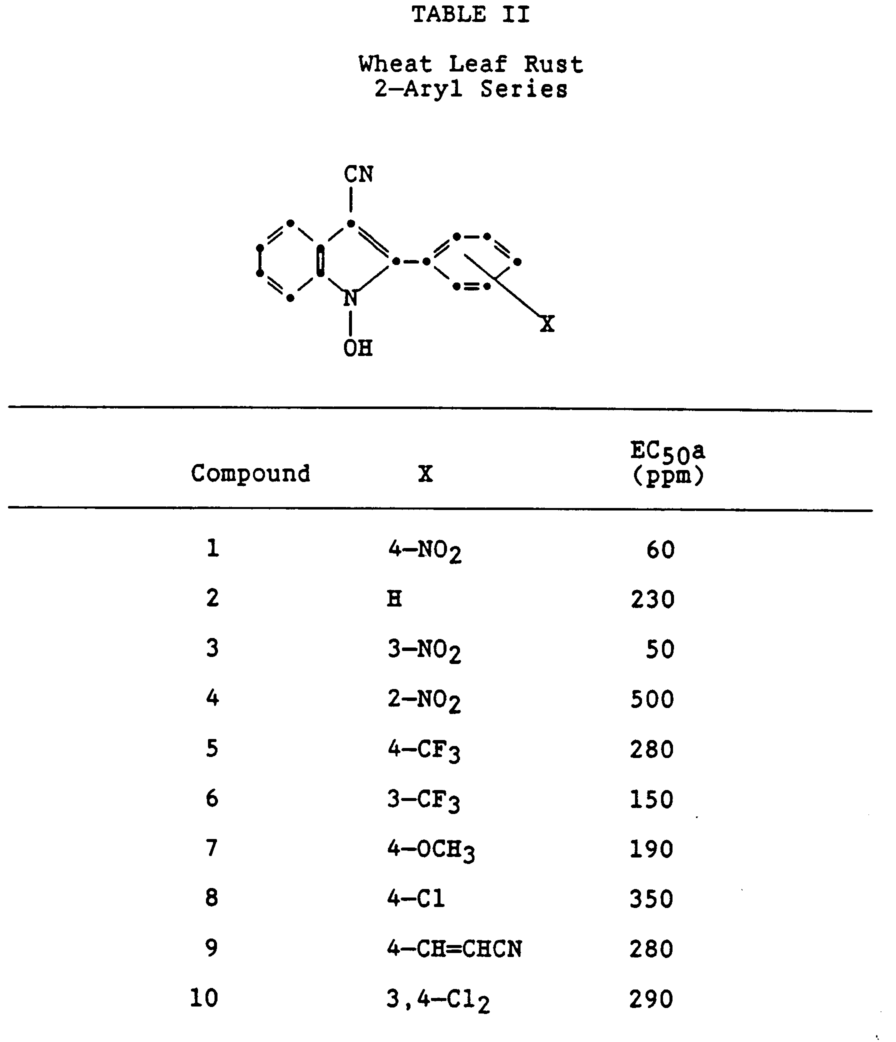

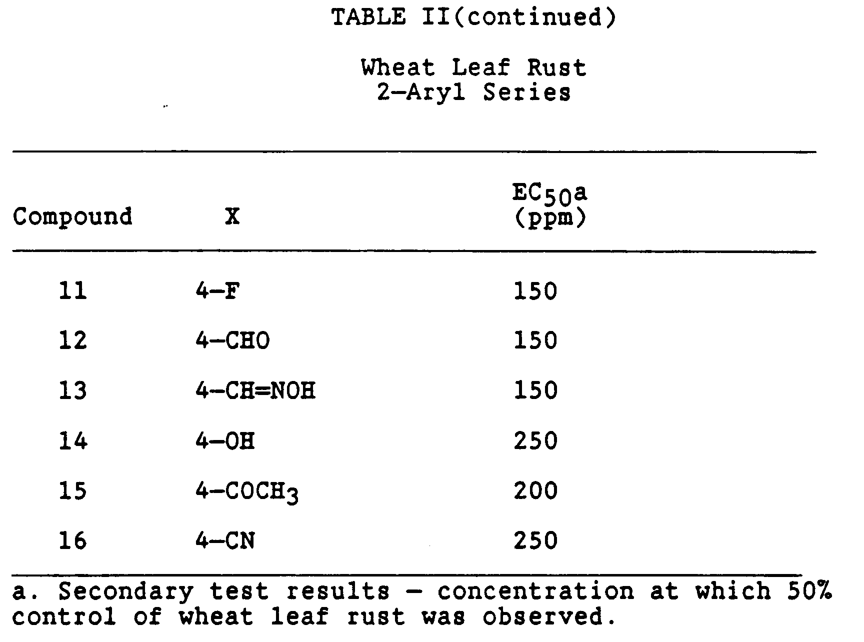

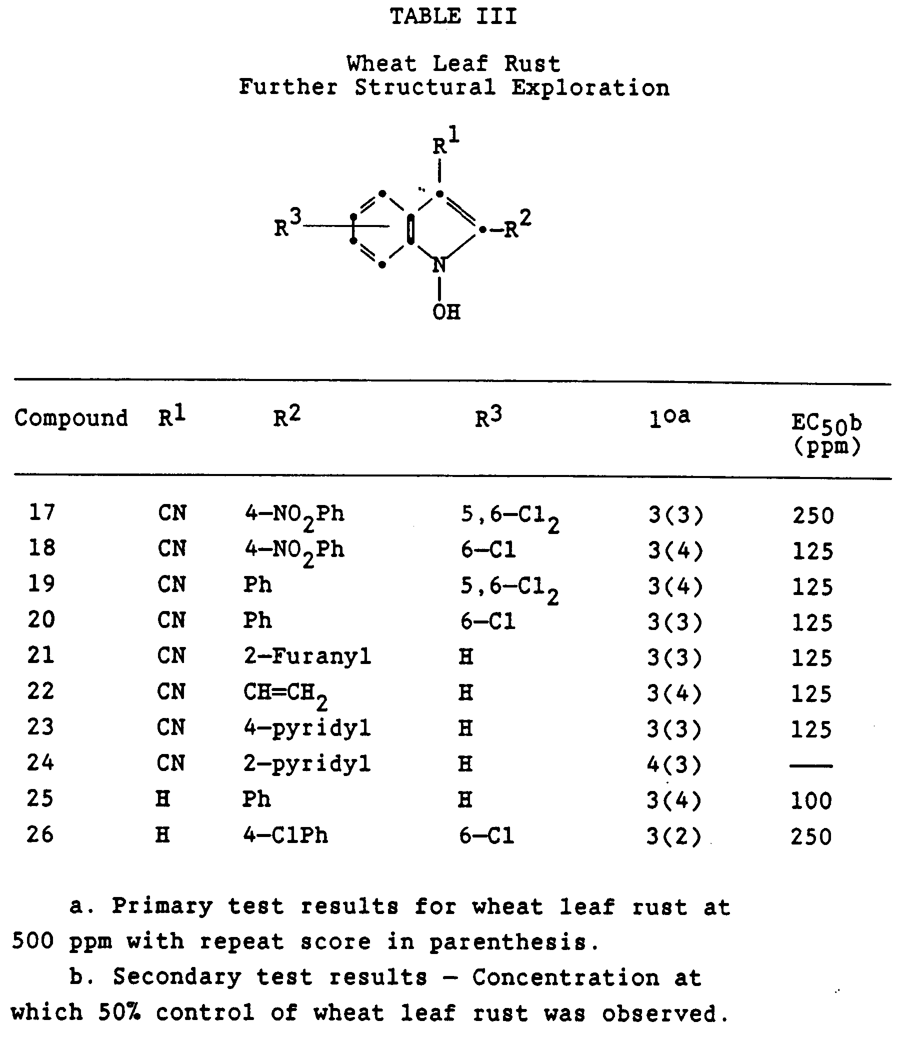

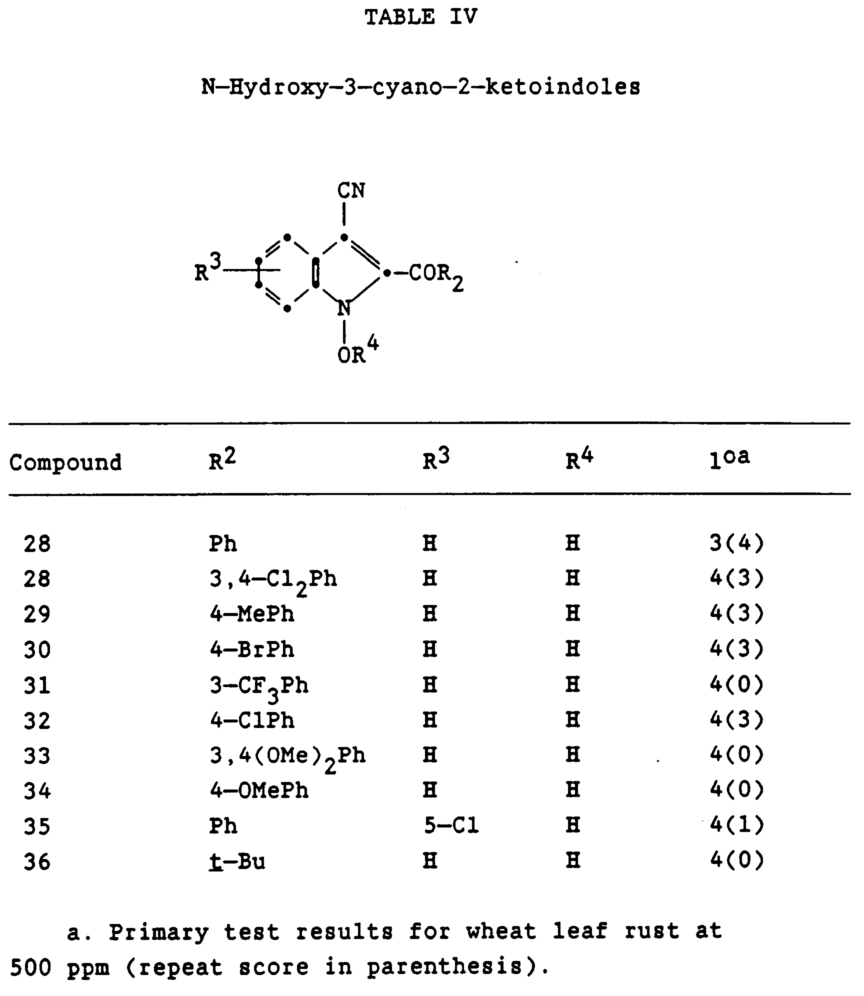

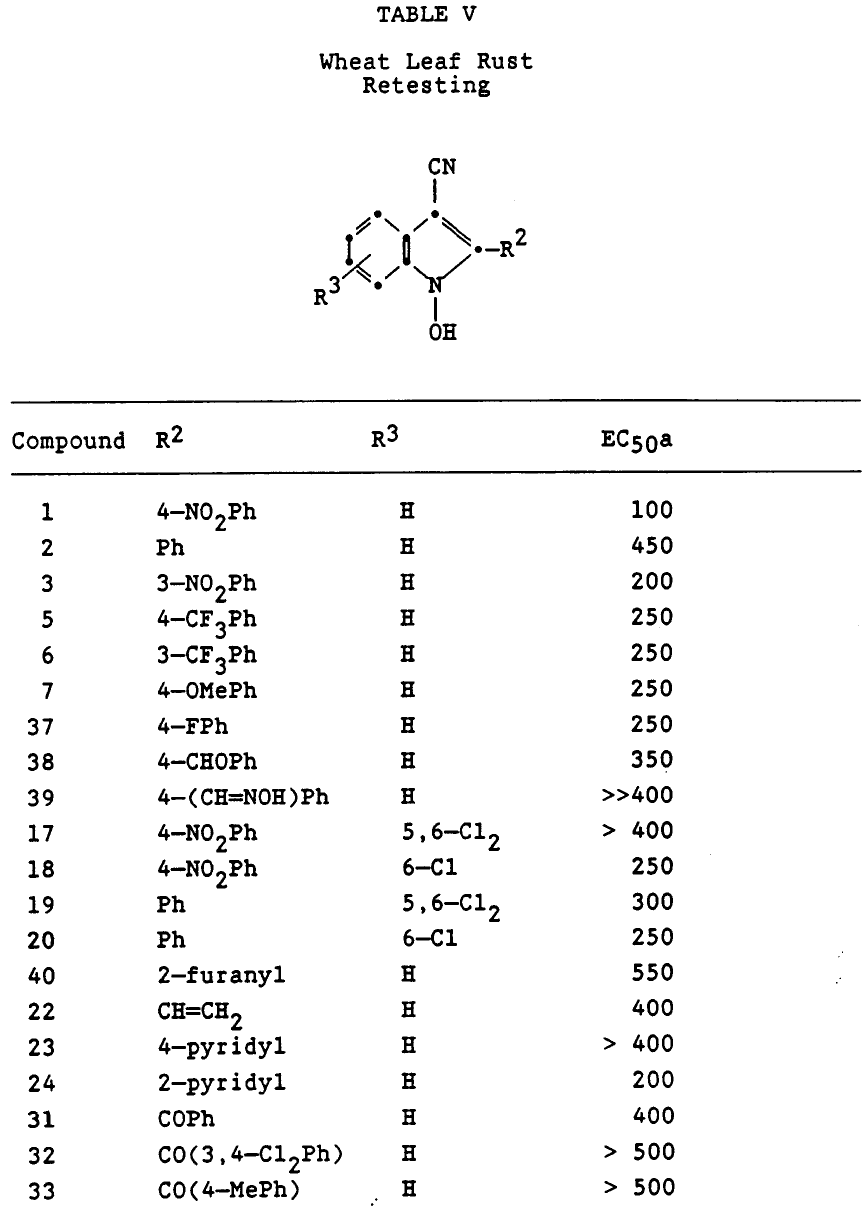

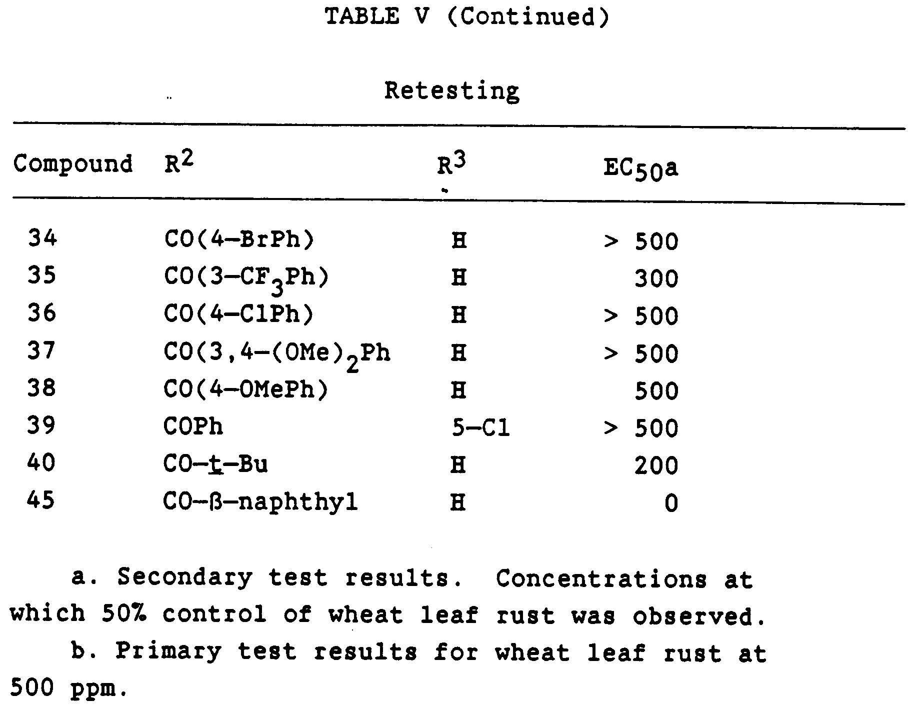

Three compounds of the invention having α-substituted-benzyl groups on the indole nucleus at the 2-position were tested for activity against Botrytis cinerea using peppers as the host (gray mold) and against Puccinia recondita using wheat as the host (wheat rust) by the following procedures:
Peppers and wheat were germinated and grown for one to three weeks (depending on species) in the greenhouse. Two pots representing two replicates of each plant species were placed in a flat such that each flat contains all the plants to be sprayed by one compound. The plants in each flat were sprayed to runoff at the rate of 135 ppm active ingredient with either the test compound or a fungicide standard. As a control, check plants were sprayed with water. The plants were allowed to air dry two to three hours. After drying, the plants were sorted and grouped by plant species.

Plant pathogenic fungus Botrytis cinerea, was grown in the laboratory on appropriate media. Inoculum from the fungus was harvested and concentrations adjusted to predetermined levels. The obligate plant pathogenic fungus, Puccinia recondita f.sp. tritici was harvested from its host in the greenhouse and concentrations were adjusted to predetermined levels.

The plants previously treated with test compounds were sprayed with fungal inoculum and then placed in humidity chambers for a period of time previously determined to be optimum for development of each disease. After incubation, the plants were moved to the greenhouse, symptoms allowed to develop (one week), and the plants evaluated for disease intensity. The data reported in Table VI is the percent disease control at 135 ppm, and represents the average of the two replicates.

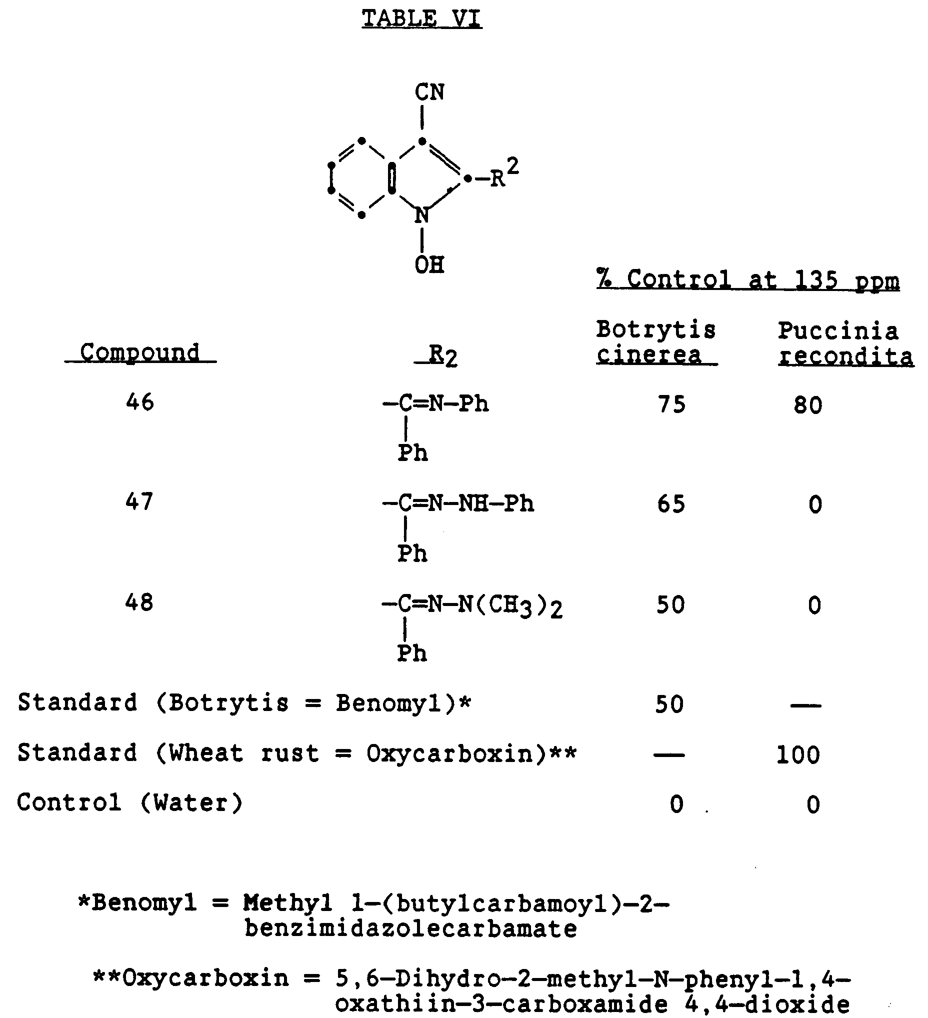

### Comparative Example 1

Four N-substituted indoles lacking an oxygen substituent in the 1-position of the indole were tested for fungicidal activity. The structure of the compound employed as the active ingredient can be seen in Table VII. These comparative compounds denoted 49-53 were evaluated for fungicidal activity against wheat leaf rust, as shown in Example 1.

As the data shown in Table VII indicate, none of the structures showed fungicidal activity.

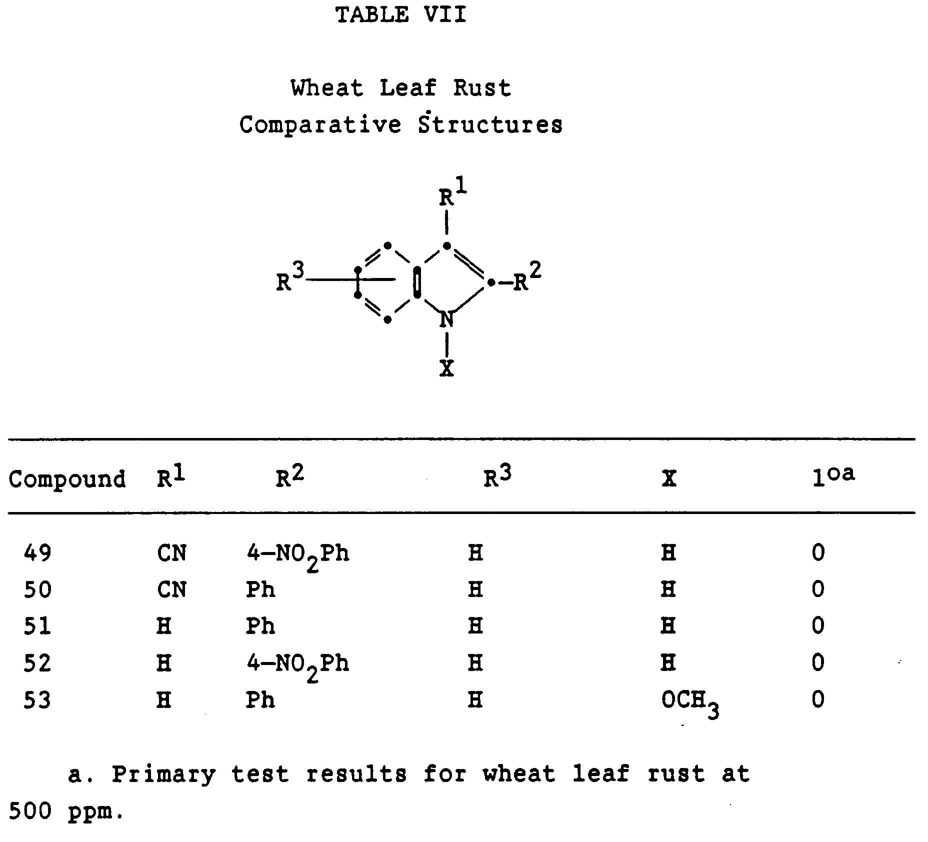

## Claims

1. A process for controlling fungus comprising contacting the fungus with an active compound
characterized wherein the compound is employed in an amount ranging from 1 to 90 weight percent and is a 1-hydroxyindole having a formula as follows: 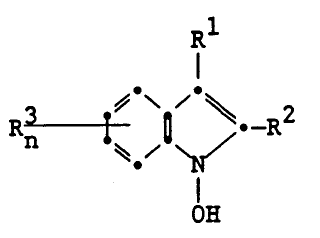 wherein:
R¹ is hydrogen, carbamoyl, t-butylcarbamoyl, dimethylcarbamoyl, carboxy, nitro, or cyano,
R² is an alkenyl having 2-10 carbon atoms, N-substituted-α-iminobenzyl, an unsubstituted or substituted aromatic group, or an acyl having 2-16 carbon atoms,
R³ is a halogen atom, and
n is an integer from 0 to 4.

2. A process according to claim 1 wherein
R¹ is cyano or hydrogen.

3. A process according to claim 1 wherein
R¹ is cyano,
R² is 4-nitrophenyl, 3-nitrophenyl, 3-trifluoromethylphenyl, 4-methoxyphenyl, 4-fluorophenyl, 4-formylphenyl, 4-carbamoylphenyl, 2-furyl, vinyl, 4-pyridyl, 2-pyridyl, phenyl, 3,4-dichlorophenyl, 4-methylphenyl, 4-bromophenyl, 4-chlorophenyl, 3,4-dimethoxyphenyl, 4-methoxyphenyl, 3-trifluoromethylbenzoyl, 4-t-butylphenyl, hydroxyiminomethylphenyl, or α-(phenylimino)benzyl, and
n is 0.

4. A process according to claim 3 wherein
R² is phenyl or 4-nitrophenyl.

5. A process according to claim 1 wherein
R¹ is hydrogen,
R² is phenyl, 4-nitrophenyl or
4-chlorophenyl,
R³ is chloro, and
n is 0 to 2.

6. A process according to any of claims 1 to 3 wherein R² is meta or para substituted phenyl group.

7. The process according to any of claims 1 to 6 wherein said fungus is wheat leaf rust.

8. A process according to any of claims 1 to 7 further comprising employing a solvent selected from acetone, methanol, ethanol, dimethylformamide, or methyl sulfoxide, wherein the solvent is extended with water.

9. A process according to any of claims 1 to 8 further comprising employing an emulsifying agent wherein the active compound is employed in an amount ranging from 10 to 90 weight percent.

10. A process according to any of claims 1 to 7 or 9 wherein the active compound is prepared as a dust and combined with a finely divided inert solid selected from botanical flours, silica, silicates, carbonates or clays.

## Patentansprüche

1. Verfahren zur Kontrolle des Pilzwachstums, bei dem man den Pilz mit einer aktiven Verbindung in Kontakt bringt,
dadurch gekennzeichnet, daß die Verbindung in einer Menge von 1 bis 90 Gew.-% verwendet wird, und ein 1-Hydroxyindol der folgenden Formel ist: 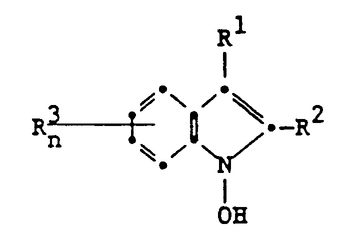 worin bedeuten:
R¹ Wasserstoff, Carbamoyl, t-Butylcarbamoyl, Dimethylcarbamoyl, Carboxy, Nitro oder Cyano,
R² eine Alkenylgruppe mit 2 bis 10 Kohlenstoffatomen, N-substituiertes α-Iminobenzyl, eine unsubstituierte oder substituierte aromatische Gruppe oder eine Acylgruppe mit 2 - 16 Kohlenstoffatomen,
R³ ein Halogenatom und
n eine Zahl von 0 bis 4.

2. Verfahren nach Anspruch 1, bei dem R¹ für Cyano oder Wasserstoff steht.

3. Verfahren nach Anspruch 1, bei dem R¹ für Cyano steht,
R² steht für 4-Nitrophenyl, 3-Nitrophenyl, 3-Trifluoromethylphenyl, 4-Methoxyphenyl, 4-Fluorophenyl, 4-Formylphenyl, 4-Carbamoylphenyl, 2-Furyl, Vinyl, 4-Pyridyl, 2-Pyridyl, Phenyl, 3,4-Dichlorophenyl, 4-Methylphenyl, 4-Bromophenyl, 4-Chlorophenyl, 3,4-Dimethoxyphenyl, 4-Methoxyphenyl, 3-Trifluoromethylbenzoyl, 4-t-Butylphenyl, Hydroxyiminomethylphenyl oder α-(Phenylimino)benzyl, und
n ist gleich 0.

4. Verfahren nach Anspruch 3, bei dem R² steht für Phenyl oder 4-Nitrophenyl.

5. Verfahren nach Anspruch 1, bei dem R¹ steht für Wasserstoff,
R² steht für Phenyl, 4-Nitrophenyl oder 4-Chlorophenyl,
R³ steht für Chloro und
n ist eine Zahl von 0 bis 2.

6. Verfahren nach einem der Ansprüche 1 bis 3, bei dem R² für eine meta- oder para-substituierte Phenylgruppe steht.

7. Verfahren nach einem der Ansprüche 1 bis 6, bei dem der Pilz ein Weizenblatt-Rostpilz ist.

8. Verfahren nach einem der Ansprüche 1 bis 7, weiter umfassend die Verwendung eines Lösungsmittels, ausgewählt aus Aceton, Methanol, Ethanol, Dimethylformamid oder Methylsulfoxid, wobei das Lösungsmittel mit Wasser verdünnt ist.

9. Verfahren nach einem der Ansprüche 1 bis 8, weiter umfassend die Verwendung eines Emulgiermittels, wobei die aktive Verbindung in einer Menge von 10 bis 90 Gew.-% verwendet wird.

10. Verfahren nach einem der Ansprüche 1 bis 7 oder 9, bei dem die aktive Verbindung hergestellt wird als Staub und kombiniert wird mit einer feinteiligen inerten festen Masse, ausgewählt aus botanischen Mehlen, Kieselsäure, Silicaten, Carbonaten oder Tonen.

## Revendications

1. Procédé pour contrôler les champignons dans lequel on met en contact le champignon avec un composé actif
caractérisé en ce que le composé est employé en quantité comprise entre 1 et 90 % en poids et est un 1-hydroxyindole ayant la formule : 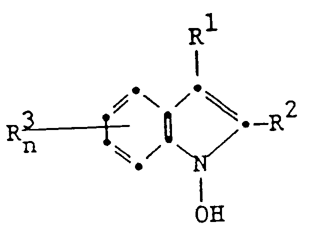 où
R¹ est l'hydrogène, carbamyle, t-butylcarbamyle, diméthylcarbamyle, carboxy, nitro, ou cyano
R² est un alcényle de 2 à 10 atomes de carbone, un α-iminobenzyle n-substitue, un groupe aromatique substitué ou non, ou un acyle ayant de 2 à 16 atomes de carbone,
R³ est un atome d'halogène, et
n est un entier de 0 à 4.

2. Procédé selon la revendication 1 dans lequel R¹ est cyano ou hydrogène.

3. Procédé selon la revendication 1 dans lequel
R¹ est cyano.
R² est 4-nitrophényle, 3-nitrophényle, 3-trifluorométhylphényle, 4-méthoxyphényle, 4-fluorophényle, 4-formylphényle, 4-carbamylphényle, 2-furyle, vinyle, 4-pyridyle, 2-pyridyle, phényle, 3,4-dichlorophényle, 4-méthylphényle, 4-bromophényle, 4-chlorophényle, 3,4-diméthoxyphényle, 4-méthoxyphényle, 3-trifluorométhylbenzyle, 4-t-butylphényle, hydroxyiminométhylphényle, ou α-(phénylimino)benzyle, et
n est O.

4. Procédé selon la revendication 3 dans lequel R² est phényle ou 4-nitrophényle.

5. Procédé selon la revendication 1 dans lequel
R¹ est l'hydrogène
R² est phényle ou 4-nitrophényle, ou
4-chlorophényle,
R³ est chloro, et
n est 0,1 ou 2.

6. Procédé selon l'une quelconque des revendications 1 à 3 dans lequel R² est un groupe phényle substitué en position méta ou para.

7. Procédé selon l'une quelconque des revendications 1 à 6 dans lequel le champignon est la rouille de la feuille de blé.

8. Procédé selon l'une quelconque des revendications 1 à 7 comprenant l'emploi d'un solvant choisi parmi l'acétone, le méthanol, l'éthanol, le diméthylformamide, le méthyl sulfoxide où le solvant est étendu avec de l'eau.

9. Procédé selon l'une quelconque des revendications 1 à 8 comprenant de plus l'emploi d'un agent émulsifiant dans lequel le composé actif est employé en quantité comprise entre 10 et 90 % en poids.

10. Procédé selon l'une quelconque des revendications 1 à 7 ou 9 dans lequel le composé actif est préparé sous forme de poussière et combiné avec un solide inerte finement divisé choisi parmi les poudres végétales, la silice, les silicates, les carbonates ou les argiles.
